# EUROPEAN PATENT APPLICATION

(11) **EP 1 972 263 A2**
(43) Date of publication of application: **24.09.2008**
(21) Application number: 08005517.1
(22) Date of filing: 25.03.2008
(51) Int. Cl.: A61B 1/05, A61B 8/12

(54) **Medical apparatus obtaining information indicative of internal state of an object based on interaction between ultrasound waves and light**

(30) Priority: 23.03.2007 JP 2007077653
(71) Applicant: OLYMPUS MEDICAL SYSTEMS CORP., Shibuya-ku Tokyo 151-0072 (JP)
(72) Inventor: Igarashi, Makoto, Tokyo 151-0072 (JP); Gono, Kazuhiro, Tokyo 151-0072 (JP)
(74) Representative: von Hellfeld, Axel

(57) **Abstract**

A biological observation system (1) is provided, which is mounted in an endoscope (2) so as to acquire information indicating the internal state of a body tissue of an object using the interaction between ultrasound waves and light. The endoscope is equipped with an ultrasound generating member (22g,22P), a light transmitting/receiving member (22i,21b,22j,22P,22l,22m), and a moving member (22e,22e1,59a,22e2,8). The ultrasound generating member generates ultrasound waves toward a region to be examined of an object. The light transmitting/receiving member radiates light from a light source toward the region to be examined and receives light reflected by a region of the ultrasound waves within the region to be examined. The moving member spatially moves both the ultrasound generating member and the light transmitting/receiving member together as a unitary member.

## Description

### CROSS REFERENCE TO RELATED APPLICATION

The patent application related to and incorporates by reference Japanese Patent Application No. 2007-077653 filed on March 23, 2007.

### Background of the Invention

### (The field of the Invention)

The present invention relates to a medical apparatus for observing the inside of an object, and in particular, to the medical apparatus that is able to obtain information from a region being examined within the body tissue of the object on the basis of the interaction between ultrasound wave and light.

### (Related art)

Various types of medical modalities have been known. An optical imaging apparatus, which has been highlighted recently, and the conventionally used endoscope are among those medical modalities. Of these medical apparatuses, the endoscope has been widely used in the medical field as well as the industrial field. Especially, in the medical field, the endoscope is used to observe the tissue in patient's body cavities and others and perform various treatments.

Meanwhile, medical modalities which obtain tomographic images of objects based on optical imaging are also known recently. The optical imaging includes various types of techniques such as optical CT (computed tomography), optical coherence CT, photo-acoustic method, and ultrasound modulated optical tomography. In the medical field, these kinds of medical imaging techniques have attracted attention as a technique to observe various symptoms in objects in terms of a simple and noninvasive manner. Additionally, a system in which an optical imaging apparatus and an endoscope are implemented in a combined manner has also been known.

The ultrasound modulated optical tomography is an imaging technique that uses interaction between the ultrasound waves and the light. In an apparatus for performing this ultrasound modulated optical tomography, ultrasound and light are radiated into the biological body of an object to cause the light to pass an in-body region in which the radiated ultrasound waves are locally condensed. The light is subjected to modulation or scattering when passing the region to generate an optical component in which the modulation or scattering is reflected. The optical component is acquired as characteristic information, on which data of tomographic images of the body inside can be obtained. By way of example, Japanese Patent Application Publication (Laid-open) No. 2000-88743 discloses an optical measurement apparatus which provides tomographic images of a body inside by using the ultrasound modulated optical tomography.

However, the optical measurement apparatus disclosed by the publication is silent about positioning the converged region of ultrasound waves, the radiating direction of light, and the direction along which a region being examined exists. Thus, when observing a desired deeper region of an object being examined, it may be insufficient to acquire necessary characteristic information, because of insufficient scanning at the desired deeper region.

### Summary of the Invention

The present invention has been made in consideration of the foregoing conventional situation, and an object of the present invention is to provide a medical apparatus that has the capability of reliably acquiring characteristic information from a desired deeper region in the membrana mucosa of the body tissue of an object.

In order to achieve the above object, the endoscope according to the present invention comprises an ultrasound generating member (22g, 22P) that generates ultrasound waves toward a region to be examined of an object; a light transmitting/receiving member (22i, 21b, 22j, 22P, 22l, 22m) that radiates light from a light source toward the region to be examined and receives light reflected by a region of the ultrasound waves within the region to be examined; and a moving member (22e, 22e1, 59a, 22e2, 8) that spatially moves both the ultrasound generating member and the light transmitting/receiving member together as a unitary member.

In addition, the medical apparatus according to the present invention comprises an ultrasound generating member (22g, 22P) that generates ultrasound waves toward a region to be examined of an object; a light source unit (4) that emits light reaching the region to be examined; a light transmitting/receiving member (22i, 21b, 22j, 22P, 221, 22m) that radiates light from a light source unit toward the region to be examined and receives light reflected by a region of the ultrasound waves within the region to be examined; and a moving member (22e, 22e1, 59a, 22e2, 8) that spatially moves both the ultrasound generating member and the light transmitting/receiving member together as a unitary member.

By way of example, the ultrasound generating member, the light transmitting/receiving member, and the moving member are arranged in the insertion tube of an endoscope.

The moving member is able to spatially move (i.e., rotate or vibrate) both the ultrasound generating member and the light transmitting/receiving member together as a unitary member. Thus, the converged region (beam-formed region) of the ultrasound waves and the radiating direction of the light are made to agree with a direction in which a region being examined exists. With this positionally matched state kept, both the ultrasound waves and the light can be radiated toward the region. It is therefore possible to reliably acquire characteristic information from a desired deeper region in the membrana mucosa of the body tissue of an object.

### Brief Description of the Drawings

In the accompanying drawings:
Fig. 1 is a block diagram showing the configuration of essential components of a biological observation system in which an endoscope is used, which is according to a first embodiment of the present invention;
Fig. 2 is a partial block diagram showing a modification of a distal end of the endoscope shown in Fig. 1;
Fig. 3 is a block diagram showing the configuration of a modified form of the biological observation system shown in Fig. 1;
Fig. 4 is a partial block diagram detailing the configuration of the distal end of the endoscope shown in Fig. 3;
Fig. 5 is a block diagram showing the configuration of essential components of a biological observation system in which an endoscope is used, which is according to a second embodiment of the present invention;
Fig. 6 is a partial block diagram detailing the configuration of the distal end of the endoscope shown in Fig. 5; and
Fig. 7 is a block diagram showing the configuration of essential components of a biological observation system in which an endoscope is used, which is according to a second embodiment of the present invention.

### Detailed Description of the Preferred Embodiments

With reference to the accompanying drawings, various embodiments of the present invention will now be described.

### (First embodiment)

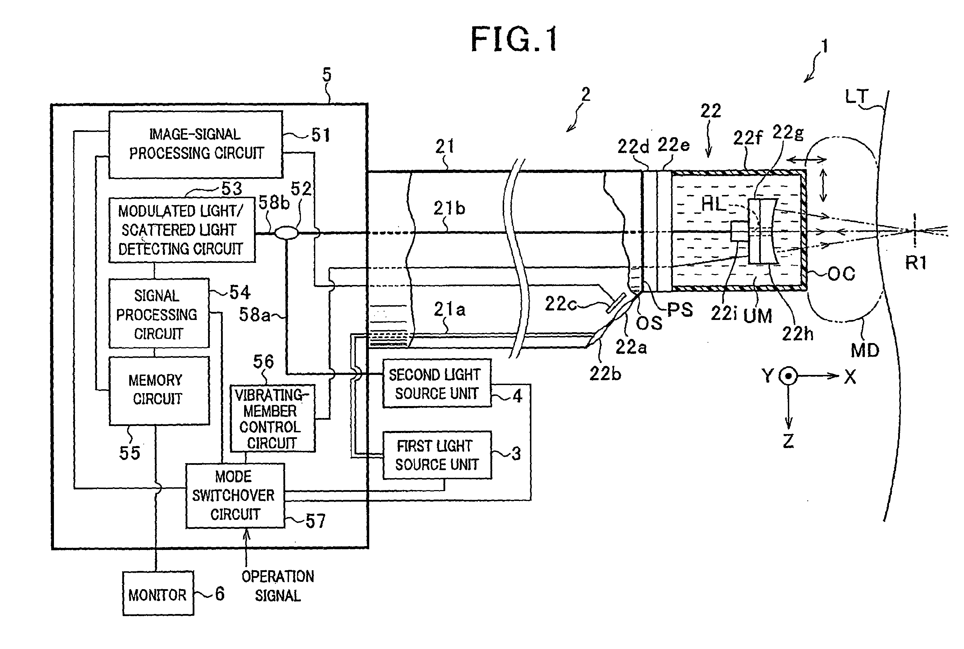

Referring to Figs. 1-4, a first embodiment of the present invention will now be described.

A biological observation system 1 according to the present embodiment is provided as the medical apparatus of the present invention. As shown in Fig. 1, the biological observation system 1 includes, as its essential components, an endoscope 2, a first light source unit 3, a second light source unit 4, a controller 5, and a monitor 6. Of these, the endoscope is insertable into a body cavity of an object being examined and is able to observe a region to be examined within a body tissue LT residing in the body cavity. The first light source unit 3 is configured to radiate illuminating light for illuminating the region to be examined in the conventional observation mode which is later described. The second light source unit 4 is configured to radiate light for observing the inside of the region to be examined in an ultrasound modulated optical tomography mode later described. The controller 5 is in charge of applying predetermined processes to electrical signals and light to be outputted from the endoscope 2 in order to produce image signals based on the electrical signals and light and output the produced image signals. The monitor 6 is configured to provide images depending on the image signals outputted from the controller 5.

The endoscope 2 is mechanically and electrically connectable with the controller 5, as shown in Fig. 1, and is equipped with an elongated and flexible insertion tube 21 which is insertable into a body cavity and a cylindrical and rigid cap device 22 secured to the distal end of the insertion tube 21. The cap device 22 may be configured such that this device 22 is detachable to the distal end of the insertion tube 21.

During the observation time, a space between the cap device 22 of the endoscope 2 and a body tissue LT (membrane mucosa, for example) is filled with a medium MD, such as water, to transmit ultrasound waves therethrough. Alternatively, the cap device 22 may be located to directly touch the body tissue LT without a gap therebetween.

In the present embodiment, as shown in Fig. 1, for the sake of convenience of the description, the XYZ orthogonal coordinate system is set such that the X-axis is assigned to the longitudinal direction (axis direction) of the cap device 22 of the endoscope 2.

In the insertion tube 21, there are provided with various signal lines (not shown) to transmit electrical signals, a first light guide cable 21a, and a second light guide cable 21b. Of these, the first light guide cable 21a is used in the conventional observation mode to transmit, to the cap device 22, light emitted from the first light source unit 3 and dedicated to illuminate a desired region to be examined. The second light guide cable 21b is used, in the ultrasound modulated optical tomography mode, to not only transmit, to the cap device 22, light (radiating light) emitted from the second light source unit 4 and dedicated to being modulated at a desired region being examined but also transmit, to the controller 5, reflected light detected by the cap device 22.

The insertion tube 21 has a distal surface which includes, as shown in Fig. 1, a surface (perpendicular surface) PS perpendicular to the X-axis and an oblique surface OS continuous from the lower edge of the perpendicular surface PS and oblique to the X-axis. On the oblique surface OS and a space near the surface OS, there are provided with an objective optical system 22a, an illuminating optical system 22b, and an image pickup device 22c. The objective optical system 22a and illuminating optical system 22b are secured to the oblique surface OS so as to have an oblique axis to an inserting direction along which the insertion tube 21 is inserted (i.e., this inserting direction corresponds to the X-axis direction when the insertion tube 21 is inserted straight). The image pickup device 22c is located at a position where images captured by the objective optical system 22a are focused.

Under the conventional observation mode, the illuminating optical system 22b receives the light emitted from the first light source unit 3 and transmitted through the first light guide cable 21a and uses the light to illuminate a desired body tissue LT. The image pickup device 22c includes, as image pickup means, a CCD (charge-coupled device), for example. This mage pickup device 22c is used to image the body tissue LT under the conventional observation mode. This device 22c produces electrical signals in accordance with images of the body tissue LT focused by the objective optical system 22a and outputs the produced electrical signals to the controller 5.

Moreover, in the cap device 22, there are provided with a vibration buffering member 22d secured on the perpendicular surface PS, a vibrator 22e secured on the vibration buffering member 22d so as to mechanically vibrate (small amounts of motion), and an ultrasound/light transmitting receiving device 22f secured on the vibrator 22e. The ultrasound/light transmitting receiving device 22f may be integrated with the cap device 22 as a part thereof or may be a separated adaptor detachably secured to the vibrator 22e.

The vibration buffering member 22d, which is made of for example rubber, is used to block off or suppress vibrations being transmitted from the vibrator 22e to the objective optical system 22a, lighting optical system 22b, and image pickup device 22c. As an example, the vibrator 22e comprises a plurality of piezoelectric elements arranged in parallel to each other to be flat, and the respective piezoelectric elements are driven as a whole or element by element in a selective manner. Thus depending on a drive signal (electrical signal) given from the controller 5, the vibrator 22e is able to mechanically vibrate (small amounts of motion) in any of the X-, Y- and Z-axis directions, that is, three-dimensionally.

The ultrasound/light transmitting receiving device 22f has a cylindrical outer casing OC with an inner space therein and, as described later, a mechanism for transmitting and receiver ultrasound waves and light is provided within the inner space of the outer casing OC. The outer casing OC is made of for example optically transmissive materials such as transmissive plastic allowing light to pass therethrough. The inner space of the outer casing OC is filled with a medium UM, such as water, to transmit ultrasound waves in order to transmit the vibration generated by the vibrator 22e to the mechanism contained in the inner space.

The ultrasound/light transmitting and receiving mechanism provided in the inner space of the outer casing includes an ultrasound transducer 22g serving as an ultrasound generator, an acoustic lens 22h serving as an ultrasound converging member, and a light transceiver 22i. The ultrasound transducer 22g employs a piezoelectric elements to mechanically vibrate (small amounts of motion) in response to an electric drive signal to be given so that the transducer is able to generate ultrasound waves.

The acoustic lens 22h is arranged as a unitary member with the ultrasound transducer 22g and beam-form (i.e., converge) the ultrasound waves emitted from the ultrasound transducer 22g. Hence the ultrasound waves emitted from the ultrasound transducer 22g are radiated to a body tissue LT via the transmission medium UM, outer casing OC, and transmission medium MD.

The light transceiver 22i is also integrated with the ultrasound transducer 22g and optically connected to one end face of the second light guide cable 21b arranged to reach and extend to and in the inside of the ultrasound/light transmitting receiving device 22f. Thus the light transceiver 22i is able to emit the light transmitted from through the second light guide cable 21b toward a region to be examined existing within the body tissue LT and to receive light reflected in the region to be examined.

In addition, to allow the light being emitted and incoming from and into the light transceiver 22i to pass without encountering any objections, a given-diameter hole is formed at a Y-Z planar central part of each of the ultrasound transducer 22g and the acoustic lens 22h.

Accordingly, since the ultrasound/light transmitting receiving device 22f has the components described above, the light emitted from the light transceiver 22i and the ultrasound waves beam-formed by the acoustic lens 22h are radiated in the same direction (in the case shown in Fig. 1, in the X-axis direction).

The ultrasound waves radiated as above has a frequency which allows the waves to be propagated in the living body and is a rarefactional wave, whereby the ultrasound waves are propagated in the body tissue LT. Of the ultrasound waves propagated in the body tissue LT, a converged region where the sound pressure is dense serves as an optically mirror. Thus the light radiated by the light transceiver 22i is reflected at the converged region having a dense sound pressure and returned as reflected light to the light transceiver 22i. In this case, the reflected light has been modulated, for example, in its frequency by interaction made between the ultrasound waves and light. Hence the frequency of the reflected light that has returned differs from that of the light radiated from the light transceiver 22i by an amount of Δf corresponding to the modulation.

The first light source unit 3 has, for example, a xenon lamp which emits white light. This light source unit 3 is operative in the conventional observation mode and configured to emit the light, serving as illuminating light for illuminating an object to be observed, to the first light guide cable 21a under the control of the controller 5.

The second light source unit 4, which serves as a light source according to the present invention, emits light (i.e., light beam) that is able to reach a region to be examined residing within the body tissue LT. This second light source unit 4 is produced to have a laser light source or an SLD (Super Luminescent Diode), for example. The second light source unit 4 is for use in the ultrasound modulated optical tomography mode and responds to control from the controller 5 so as to emit the light (radiating light) to a light cable 58a. The radiated light passes the controller 5 by way of the light guide cable 58a later described to the second light cable 21b.

The controller 5, serving as an image signal producer, includes an image-signal processing circuit 51, a light coupler 52, a modulated light/scattered light detecting circuit 53, a signal processing circuit 54, a memory circuit 55, a vibrating-member control circuit 56, a mode switchover circuit 57, and light guide cables 58a and 58b, as shown in Fig. 1.

The image-signal processing circuit 51 is under the control of the mode switchover circuit 57 and produces video signals depending on image pickup signals coming from the image pickup device 22c of the endoscope 2 and outputs the produced video signals to the memory circuit 55.

The light coupler 52 is optically connected to both the second light guide 21b and the light guide cables 58a and 58b arranged in the controller 5. This light coupler 52 receives light emitted from the second light source unit 4 and transmitted through the light guide cable 58a, and sends out the received light to the second light guide cable 21b. Meanwhile, reflected light coming through the second light guide cable 21b is sent to the modulated light/scattered light detecting circuit 53 via the light coupler 52 and the light guide cable 58b. In other words, the light coupler 52 functions as a light circulator.

The modulated light/scattered light detecting circuit 53 has a not-shown oscilloscope or a spectrum analyzer. This circuit 53 detects the reflected light (modulated light) coming via the light transceiver 22i, second light guide cable 21b, light coupler 52, and light guide cable 58b, and provides the signal processing unit 54 with an electrical modulated signal in accordance with the reflected light.

The signal processing circuit 54 receives the modulated signal from the detecting circuit 53 and estimates modulation characteristic information and/or scattering characteristic information (modulation/scattering characteristic information) of the light in a local region within the body tissue LT, the local region including a portion where the light is emitted from the light transceiver 22i. This signal processing circuit 54 produces video signals based on the estimated modulation/scattering characteristic information, and outputs the video signals to the memory circuit 55.

The memory circuit 55 temporarily memorizes either the video signals outputted from the image-signal processing circuit 51 in the conventional observation mode or the video signals outputted from the signal processing circuit 54 in the ultrasound modulated optical tomography mode. While memorizing the video signals, this circuit 55 also outputs the video signals to the monitor 6 sequentially, frame by frame.

The vibrating-member control circuit 56 is in charge of controlling the vibrating state of the vibrator 22e to change a scanned position within the body tissue LT in a two-dimensional controlled manner. The scanned position is a portion of the body tissue LT where sound pressure due to the ultrasound waves propagated in the body tissue LT is dense.

The controller 5 responds to operations from a not-shown operation panel or others at which an operator is allowed to manually give operation commands. In response to such operations, the mode switchover circuit 57 selects, as the observation mode of the present biological observation system 1, either the conventional observation mode or the ultrasound modulated optical tomography mode.

Practically, when the observation mode is switched to the conventional observation mode in response to the operator's command, the mode switchover circuit 57 enables the operations of the first light source unit 3 and image-signal processing circuit 51 and disables the operations of the second light source unit 4, signal processing circuit 54, and vibrating-member control circuit 56. Thus in the conventional observation mode, the light emitted from the first light source unit 3 is used to illuminate the body tissue LT and the image pickup device 22c picks up the images of the body tissue LT.

In contrast, when the observation mode is switched to the ultrasound modulated optical tomography mode in response to the operator's command, the mode switchover circuit 57 enables the operations of the second light source unit 4, signal processing circuit 54, and vibrating-member control circuit 56. Concurrently the circuit 57 disables the operations of the first light source unit 3 and image-signal processing circuit 51. Thus in the ultrasound modulated optical tomography mode, the light (light beam) emitted from the light transceiver 22i and the ultrasound waves beam-formed by the acoustic lens 22h are radiated together to the body tissue LT.

The operations and advantages of the biological observation system 1 will now be described.

When the present system 1 is set to the conventional observation mode, the mode switchover circuit 57 enables the first light source unit 3 and the image-signal processing circuit 51 to be active as stated. Thus, the light from the first light source unit 3 travels through the first light guide cable 21a and is radiated toward a body tissue LT by the illuminating optical system 22b.

In the field of view of the objective optical system 22a, the image pickup device 22c picks up images of the body tissue LT to output image signals. Image signals are produced into video signals by the image-signal processing circuit 51, and the video signals are sent to the memory circuit 55.

The video signals from the processing circuit 51 are temporarily memorized by the memory circuit 55, during which the video signals are sent to the monitor frame by frame, in sequence. Therefore the monitor 6 uses the video signals to represent images of the body tissue LT for visual observation.

In contrast, when the observation mode of this system 1 is switched to the ultrasound modulated optical tomography mode, the second light source unit 4, signal processing circuit 54, and vibrating-member control circuit 56 are made active by the mode switchover circuit 57. Hence, the light from the second light source unit 4 travels along the light guide cable 58a, light coupler 52, and second light guide cable 21b, and is radiated toward the body tissue LT by the light transceiver 22i.

The vibrating-member control circuit 56 is already under operation by responding to the command from the mode switchover circuit 57. Thus, by this control circuit 56, the vibrator 22e is forced to two-dimensionally vibrate (small amounts of vibration) in both the X-axial and Z-axis directions (refer to Fig. 1).

When the vibrator 22e is made to spatially vibrate, the ultrasound transducer 22g that generates the ultrasound waves is also obliged to vibrate together (synchronously) with the vibrator 22e. As a result, the beam-formed position of the ultrasound waves given by the acoustic lens 22h, that is, the converged region of the ultrasound waves, is moved, for example, in the X-axis and Z-axis directions within the body tissue LT. This movement is accordance with the widths of vibration of the vibrator 22e in both the X-axial and Z-axis directions.

In the endoscope 2 of the present embodiment, the ultrasound transducer 22g, acoustic lens 22h, and light transceiver 22i are integrated in the ultrasound/light transmitting receiving device 22f. This makes it possible that the radiating direction of the light emitted toward the body tissue LT and the converged region of the ultrasound waves (i.e., the region of a dense sound pressure) are positionally matched to each other. Hence the ultrasound waves and light are scanned in, for example, an X- and Z-axial two-dimensional area within the body tissue LT in accordance with the widths of vibration of the vibrator 22e.

As shown in Fig. 1, the ultrasound waves propagated in the X-axis direction within the body tissue LT is made to converge (beam-formed), as shown by chain double-dashed lines, so as to produce a region R1 whose sound pressure is denser. In Fig. 1, the region R1 is able to be function as an optical mirror. The light transmitted through the second light guide cable 21b and emitted toward the body tissue LT is made to travel as shown by a chain line. Accordingly, during the travel, the radiated light is subjected to modulation on the interaction between the ultrasound waves and the light and reflects (at least partly reflects) at the optical-mirror region R1 having a denser sound pressure. The reflected light (modulated light) is returned to the light transceiver 22i.

The reflected light returned to the light transceiver 22i is transmitted to the modulated light/scattered light detecting circuit 53 via the second light guide cable 21b, light coupler 52, and light guide cable 58b.

As described, by the detecting circuit 53, the reflected light is detected, and sent the detected reflected light to the signal processing circuit 54 as an electrical modulated signal.

In this signal processing circuit 54, the modulated signal undergoes, for example, a process such as Fourier conversion so as to estimate modulation/scattering characteristic information in the position (region R1) within the body tissue LT, where the radiating light is reflected. In addition, video signals are produced based on the estimated modulation/scattering characteristic information, and sent to the memory circuit 55.

The video signals sent to the memory circuit 55 are temporarily stored therein, during which the signals are outputted in sequence, frame by frame, to the monitor 6. Thus, on the monitor 6, two-dimensional tomographic images are rendered which show the estimated modulation/scattering characteristic information in the scanned area included the region R1 within the body tissue LT.

As stated above, in the present biological observation system 1 that uses the endoscope 2, the configuration is kept relatively simple, but it is possible to radiate both the ultrasound waves and the light in a manner that the converged region of the ultrasound waves and the radiating direction of the light are always matched positionally to each other. In consequence, compared to the conventional, the characteristic information can be obtained reliably from a wider area in a desired deeper region within the membrane mucosa of the body tissue.

### (Modifications)

Various modifications of the first embodiment will now be described. In the modifications, for the sake of a simplified explanation, the identical or similar components to those in the first embodiment will be given the same reference numerals as those in the first embodiment.

### (First modification)

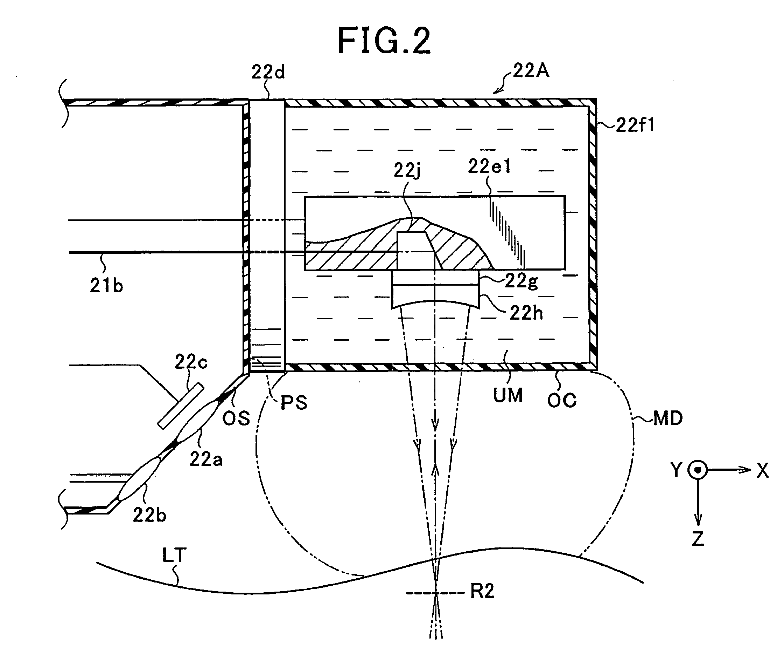

Referring to Fig. 2, a first embodiment will now be described.

The modification concerns with the radiating direction of the light and ultrasound waves which are radiated in the ultrasound modulated optical tomography mode.

In the foregoing first embodiment, the cap device 22 of the endoscope 2 is configured such that, in the ultrasound modulated optical tomography mode, both the light and the converged (beam-formed) ultrasound waves are radiated in the direction (i.e., X-axis direction) parallel with an inserting axis (imaginary axis) of the insertion tube 21. But this is not always a decisive geometry. For example, the cap device 22 may be structured to have both the light and the converged ultrasound waves radiated in the direction (i.e., the Z-axis direction) perpendicular to the inserting axis of the insertion tube 21 in the ultrasound modulated optical tomography mode. This structure is realized as shown in Fig. 2, where there is provided a cap device 22A.

As shown in Fig. 2, the cap device 22A is provided with an ultrasound/light transmitting receiving device 22f1 instead of the foregoing ultrasound/light transmitting receiving device 22f.

The ultrasound/light transmitting receiving device 22f1 is equipped with a vibrator 22e1 comprising a rectangular- parallelepiped piezoelectric element in which a light reflection member 22j consisting of a prism is embedded, in addition to the ultrasound transducer 22g and the acoustic lens 22h.

The light reflection member 22j functions as a light transceiver and is embedded in the vibrator 22e1 to level the Z-axial lower face (facing in the radiating direction) of the vibrator 22e1 at that of the member 22j. Thus, light entering this light reflection member 22j is reflected in the Z-axis direction without being blocked by the vibrator 22e1. The light reflection member 22j receives the light transmitted through the second light guide cable 21b in the inserting axis direction (X-axis direction) of the endoscope 2 and reflects this light in the perpendicular direction (Z-axis direction) to the inserting axis direction.

When the vibrator 22e1 vibrates, the ultrasound transducer 22g also vibrates together which generates the ultrasound waves. The acoustic lens 22h converges (beam-forms) the ultrasound waves radiated from the ultrasound transducer 22g in the Z-axis direction. The thus-converted ultrasound waves are radiated in a region being examined, depending on the widths of vibration of the vibrator 22e1.

Since the ultrasound/light transmitting receiving device 22f1 is configured as above, the light reflected by the light reflection member 22j and the ultrasound waves converged by the acoustic lens 22h are radiated together in the same direction (in the Z-axis direction in Fig. 2).

The radiated ultrasound waves are converged as shown by chain doubled-dashed lines in Fig. 2, in which a denser sound pressure is produced as a region R2 within the body tissue LT. The region R2, functions as a mirror for light. In contrast, the light (light beam) transmitted by the second light guide cable 21b and radiated in the Z-axis direction passes as shown by dashed lines in Fig. 2, and is reflected by the region R2 serving as a mirror to produce reflected light that returns to the light reflection member 22j. The returned reflected light is reflected by this member 22j in the X-axis direction to be transmitted to the controller 5 via the second light guide cable 21b.

In this way, the radiation of the ultrasound waves and the radiation and detection of the light are performed in parallel to each other at intervals, while the vibration 22e1 is vibrated by smaller amounts of displacement in predetermined directions (for example, at least two directions among the X-, Y- and Z-axis directions). This makes it possible to scan the ultrasound waves and the light in the region inside the body tissue LT. The scanned region is decided depending on the widths of vibration in each vibrating direction of the vibrator 22e1.

The controller 5 applies, to the detected reflected light, the processes which are similar to those in the foregoing embodiment.

As stated, the endoscope 2 has the above-described cap device 22A, so that it is possible to obtain the modulation/scattering characteristic information from the body tissue LT which resides in the field of view of the objective optical system 22a.

It is therefore possible that the present modification provides the similar operations and advantages to those in the first embodiment. In addition, the observer is able to observe the body tissue LT in a finer manner.

Incidentally the ultrasound/light transmitting receiving device 22f1 may be integral with the cap device 22A or may be formed as an adaptor detachable to the cap device 22A.

### (Second modification)

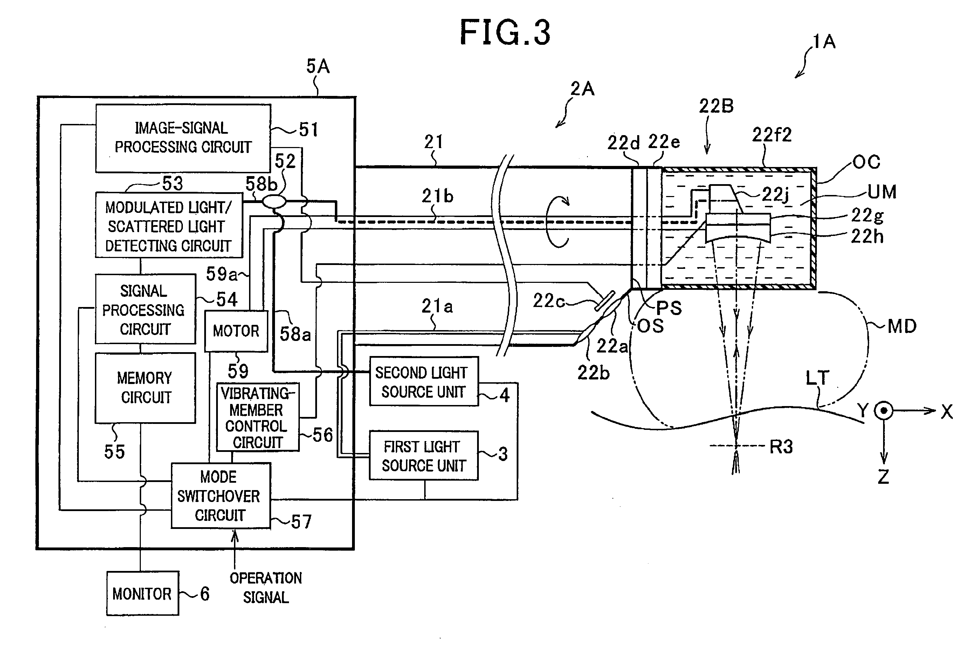

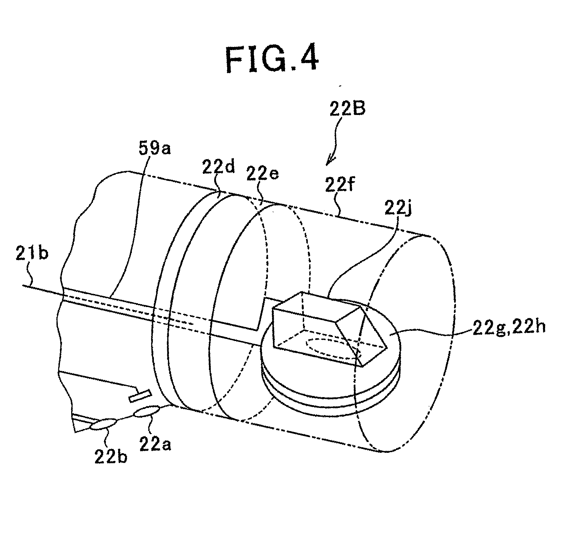

Refereeing to Figs. 3 and 4, a second modification will now be described.

The present modification also concerns the radiating directions of the light and ultrasound waves radiated in the ultrasound modulated optical tomography mode.

The biological observation system serving as a medical apparatus according to the present invention will not be limited to systems for observing tomographic images of a body tissue residing in the inserting axis direction of the endoscope. By having the configuration that allows the radiating directions of both the ultrasound waves and the light, it is possible to observe tomographic images of tubular body tissue which resides in the circumferential direction of the large bowel or others.

Fig. 3 shows a biological observation system 1A for such an application. This biological observation system 1A is provided with an endoscope 2A and a controller 5A in addition to the first and second light source units 3, 4 and the monitor 6. The endoscope 2A is equipped with a cap device 22B with an ultrasound/light transmitting receiving device 22f2, which is a substitution for the ultrasound/light transmitting receiving device 22f shown in Fig. 1. The controller 5A includes a motor 59 in addition tot the components of the controller 5 shown in Fig. 1.

The motor 59 and the ultrasound/light transmitting receiving device 22f2 are connected to each other by a flexible shaft 59a arranged in parallel with the inserting axis direction of the endoscope 2A. Part (or the whole) of the second light guide cable 21b is arranged through the flexible shaft 59a.

As shown in Fig. 4, the cap device 22B has an ultrasound/light transmitting receiving device 22f2. This device 22f2 comprises, as shown in Fig. 3, the ultrasound transducer 22g, acoustic lens 22h, and light reflection member 22j, which are formed as an integral one unit. One end of the flexible shaft 59a is mechanically connected to this unit, that is, the ultrasound transducer 22g, acoustic lens 22h, and light reflection member 22j.

The motor 59 is activated under the control of the mode switchover circuit 57. This motor is used to rotate the flexible shaft 59a in the ultrasound modulated optical tomography mode. This rotation allows the unit (the ultrasound transducer 22g, acoustic lens 22h, and light reflection member 22j) to rotate on the central longitudinal axis of the shaft 59a inside the ultrasound/light transmitting receiving device 22f2. Thus, during this rotation of the flexible shaft 59a, the radiating light reflected by the light reflection member 22j and the converged ultrasound waves by the acoustic lens 22h are radiated together in radial directions extending radially from the inserting axis of the endoscope 2.

When the light and the ultrasound waves are radiated in the Z-axis direction in Fig. 3, the radiated light travels along a dashed line, while the ultrasound waves are converged (beam-formed) as shown by chain double-dashed lines to produce a region R3 that presents a denser sound pressure within the body tissue LT. Like the foregoing ones, the region R3 becomes a mirror for the light. When the light passes in the ultrasound waves, especially, in their converged regions, the light undergoes modulation in terms of its physical characteristics on the basis the interaction between the ultrasound waves and the light. Hence the modulated light reflects at the region R3 and returns to the light reflection member 22j as the reflected light. The thus-returned reflected light is then reflected in the X-axis direction by the member 22j so as to travel through the second light guide cable 21b. The reflected light is thus transmitted to the controller 5A, where the reflected light is subjected to the same processes as those explained already.

During the rotation of the flexible shaft 59a, the above-described processing is carried out at predetermined angular intervals in the circumferential direction around the X-axis. That is, at each angular position, the light/ultrasound radial scanning is made to acquire the modulation/scattering characteristic information in relation to the body tissue.

In the present modification, it is therefore possible to have the similar operations and advantages to those in the first embodiment. Additionally, it is also possible to observe tomographic images of tissue residing in the circumferential direction of tubular body tissues including the large bowel. In particular, without positionally changing the endoscope 2A, the observer is able to visually see tomographic images acquired from wider view ranges.

By the way, the ultrasound/light transmitting receiving device 22f2 may be formed to be integral with the cap device 22B or may be formed as an adaptor detachable to the dap device 22B.

### (Second embodiment)

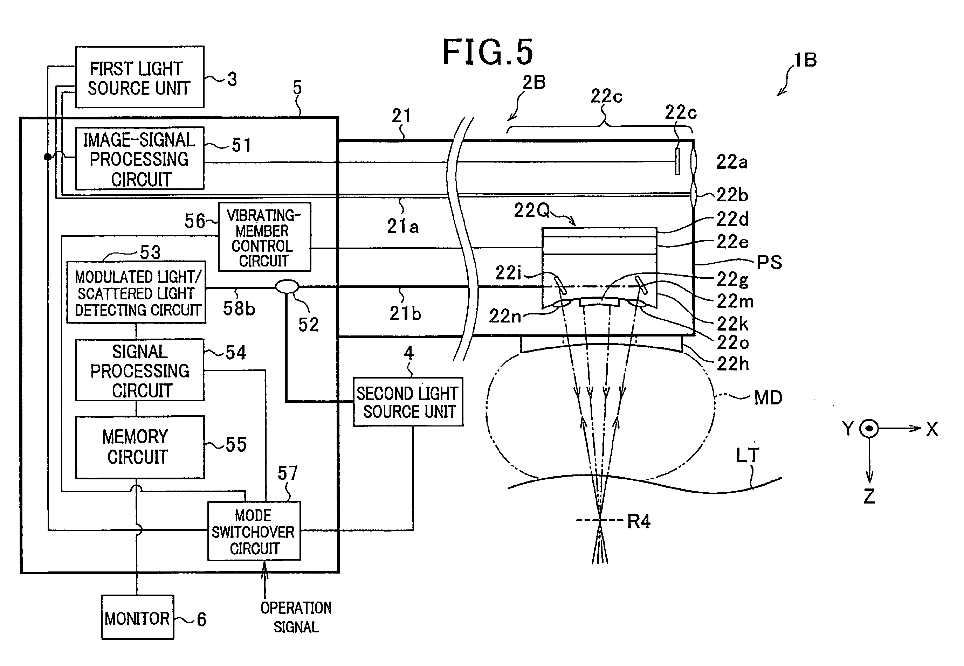

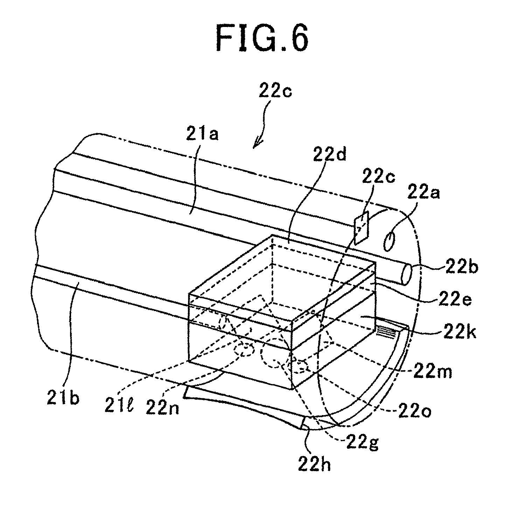

Referring to Figs. 5 and 6, a biological observation system, that is, a medical apparatus according to a second embodiment of the present invention will now be descried.

For sake of a simplified description and avoiding redundant descriptions, the similar or identical complements to those in the first embodiment or modifications thereof will be given the same reference numerals as those already described.

Fig. 5 outlines a biological observation system 1B according the second embodiment. As shown therein, this biological observation system 1B comprises an endoscope 2B, in addition to the first and second light source units 3 and 4, controller 5, and monitor 6, as its essential parts. The endoscope 2B is insertable into a body cavity of an object being examined and is used to observe a region to be examined within body tissue LT which resides in the body cavity.

As shown in Fig. 5, the endoscope 2B comprises an insertion tube 21 and a distal end 22C located at the distal portion of the insertion tube 21. This distal end 22C is equipped with a transmitting/receiving device 22Q.

The distal end 22C has a distal face PS perpendicular to the longitudinal direction (i.e., the X-axis direction) of the insertion tube 21. On the distal face PS, the objective optical system 22a and the illuminating optical system 22b are provided so as to have light axes in parallel with the longitudinal direction. At a position where images of the objective optical system 22a are focused in the distal end, the image pickup device 22c is disposed. The optical components 22a, 22b and 22c are the same as those in the first embodiment.

The transmitting/receiving device 22Q comprises a support member 22k in addition to the vibration buffering member 22d and vibrator 22e, which are stacked on one another in the order as shown to produced a unitary member. The device 22Q still comprises the ultrasound transducer 22g secured on the support member 22k. Moreover, the acoustic lens 22h is arranged at a position of the outer circumferential surface, which position faces the ultrasound output surface of the ultrasound transducer 22g. Fig. 6 shows a perspective view of this transmitting/receiving device 22Q.

The support member 22k is a member serving as a light transmitting/receiving member and made of, for example, a transmissve resin. This member 22k contains a half mirror 221 and a total reflection mirror 22m. The support member has an incident surface on which collecting lenses 22n and 220 which serve as a light converging member converging incident light.

The half mirror 22l, which receives the radiating light that has been transmitted through the second light guide cable 21b, emits part of the received light toward the collecting lens 22n by reflection and emits part of the remaining light toward the total reflection mirror 22m by transmission. The total reflection mirror 22m reflects the light which has transmitted through the half mirror 22l so as to emit the light toward the other collecting lens 220.

Both the support member 22k and the acoustic lens 22h are produced such that the incoming and outgoing light to and from the collecting lens 22n and 220 is allowed to pass therethrough without any obstruction. By way of example, the support member 22k and the acoustic lens 22h have not-shown holes formed along the light paths. Additionally, a transmissive maternal (not shown) such as transmissive resin is fit in the not-shown hole formed through the acoustic lens 22h, in order to prevent foreign matters from coming into the inside of the endoscope 2B.

All the half mirror 22l, total reflection mirror 22m, collecting lenses 22n and 220, and acoustic lens 22h are positioned to realize a situation where the radiated light passes through the ultrasound converged region, that is, both the ultrasound converged region and the light radiating direction are in the same direction.

In the present embodiment, the collecting lenses 22n and 220 may be solid lenses made of resin or glass or may be liquid lenses whose refraction index can be controlled by applying voltage thereto.

The other configurations including the controller 5 are identical or similar to those in the foregoing embodiments.

Then operations and advantages of the biological observation system 1B will now be described.

When being set to the conventional observation mode, this system 1B operates in the similar way to that in the first embodiment under the control of the controller 5. Thus the monitor 6 represents surface images of the body tissue LT for visual observation.

Meanwhile, when being switched to the ultrasound modulated optical tomography mode, the biological observation system 1B activates, as stated before, the second light source unit 4, signal processing circuit 54, and vibrating-member control circuit 56, under the control of the mode switchover circuit 57. Hence, the light (light beam) emitted from the second light source unit 4 is sent to the support member 22k via the light guide cable 58a, light coupler 52, and the second light guide cable 21b. The light propagated to the support member 22k is collected and converged by the cooperative actions of the half mirror 221, total reflecting mirror 22m, and collecting lenses 22n and 220, and radiated onto the body tissue LT. This radiated light is propagated into the body tissue LT.

During this light radiation, the vibrating-member control circuit 56 is made to vibrate, by predetermined amounts of vibration, the vibrator 22e in for example the X- and Z-axis directions (refer to Fig. 5). Thus the vibrator 22e vibrates two-dimensionally, for example.

The two-dimensional vibration of the vibrator 22e also urges the ultrasound transducer 22g to vibrate together with the vibrator 22e. The ultrasound waves, which have been radiated from the ultrasound transducer 22g and converged (beam-formed) by the acoustic lens 22h, is propagated in Z-axis direction inside the body tissue LT, where the converged region is changed two-dimensionally inside the body tissue.

Accordingly, like the first embodiment, the transmitting/receiving device 22Q is able to ultrasound-can a region being examined inside the body tissue LT in accordance with the widths of vibration o the vibrator 22e.

In the example shown in Fig. 5, the ultrasound waves radiated in the Z-axis direction is subjected to convergence shown by chain double-dashed liens and produces, as an optical mirror, a representatively depicted region R4 having a denser sound pressure. Concurrently, the light collected by the collecting lenses 22n and 220 passes along paths shown by dashed lines in Fig. 5, and then reflects (at least partly reflect) at the region R4. The reflected light returns to the collecting lenses as light which has been modulated on the interaction between the ultrasound waves and the light.

The reflected light returning toward the one collecting lens 22n is reflected by the half mirror 22l, and then emitted to the second light guide cable 21b. Meanwhile the reflected light returning toward the collecting lens 220 is reflected by the total reflection mirror 22m, passed through the half mirror 221, and emitted to the second light guide cable 21b. Thus, the reflected light travels through the second light guide cable 21b, light coupler 52, and light guide cable 58b, and sent to the controller 5.

In the controller 5, the modulated light/scattered light detecting circuit 53 applies the same process described before, to the reflected light which has been acquired. The other processes are the same in the first embodiment. Accordingly, depending on modulation/scattering characteristic information acquired from the scanned ration including the region R4 inside the body tissue LT, tomographic images are displayed on the monitor 6 as two-dimensional images, for instance.

As a result, in the biological observation system 1B according to the present embodiment, the similar advantages to that in the first embodiment can be obtained. Besides, the transmitting/receiving device 22Q is employed to converge the light from the second light guide cable 21b by combining the mirrors 22l and 22m and the collecting lenses 22n and 220. Thus the degree of freedom for designing arrangement of the light collecting elements can be raised. Thus, compared to the first embodiment, the characteristic information can be obtained reliably from a wider area in a desired deeper region within the membrane mucosa of the body tissue.

### (Third embodiment)

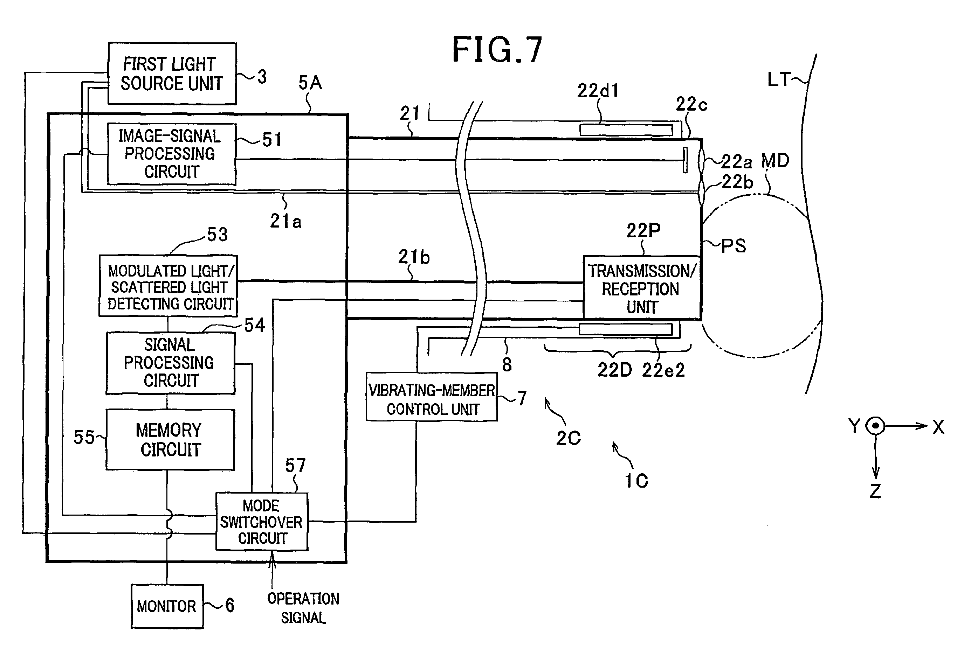

Referring to Fig. 7, a biological observation system according to a third embodiment of the present embodiment will now be described.

Fig. 7 shows a biological observation system 1C according to the present embodiment. As shown, this system 1C comprises an endoscope 2C, a controller 5A in charge of controller the entire system and producing images, a vibrating-member control unit 7 and a sheath 8, in addition to the first light source unit 3 and the monitor 6. The endoscope 2C is used in the same way as the foregoing ones. The vibrating-member control unit 7 is similar in construction to the vibrating-member control unit 56 used in the first embodiment.

As shown in Fig. 7, the endoscope 2C comprises an insertion tube 21 electrically and optically connectable to the controller 5A and a distal end 22D located at the distal section of the insertion tube 21. In the ultrasound modulated optical tomography mode, the medium MD to transmit the ultrasound waves is placed between the endoscope 22D of the endoscope 2 and the body tissue LT. Instead, the distal end 22D may be touched directly to the body tissue LT without leaving a space therebetween.

Like the second embodiment, the distal face PS of the distal end 22D is provided with the objective optical system 22a and the illuminating optical system 22b. The image pickup device 22c is arranged in the same manner at above. A transmission/reception unit 22P is arranged in the distal end 22D.

The transmission/reception unit 22P is equipped with, though not shown, a light source including a semiconductor laser, a light reception element composed of a photodiode, a ultrasound transducer, and an acoustic lens. The ultrasound transducer is formed in the same manner as the foregoing one 22g and the acoustic lens 22h is formed in the same manner as the foregoing one 22h. The transmission/reception unit 22P is under the control of the mode switchover circuit 57 in the ultrasound modulated optical tomography mode and is formed to radiate, in the X-axis direction, light (light beam) emitted from a not-shown light source toward the body tissue LT and receive reflected light from the inside of the body tissue LT by a not-shown light receiving element. In addition, this unit 22P is formed to send the received reflected light to the second light guide cable 21b.

Concurrently the transmission/reception unit 22P includes a ultrasound transducer which generates ultrasound waves and an acoustic lens which converges the generated ultrasound waves directed toward the body tissue LT.

The controller 5A is identical to the controller explained in the first embodiment except that the light coupler 52 and the vibrating-member control circuit 56 are removed from the construction.

The sheath 8, which is made of soft and flexible material, is detachably loaded to the outer surface of the insertion tube 21, including the distal end 22D, of the endoscope 2C. Thus the sheath 8 is shaped into an elongated cylindrical form that can cover the insertion tube 21, including the distal end 22D. Within the inner space of the sheath 8, there are provided a vibration buffering member 22d1 and a vibrator 22e2 at positions facing the outer sides of the distal end 22D. The vibration buffering member 22d1 is produced in the similar manner to the foregoing vibration buffering member 22d. The vibrator 22e2 is also produced similarly to the foregoing vibrator 22e and is able to vibrate three-dimensionally (i.e., in any of the X-, Y- and Z-axis directions) in response to a drive signal from the vibrating-member control unit 7. In addition, both the member 22d1 and the vibrator 22e2 are curved along the outer side surface of the distal end 22D.

The operations and advantages of the biological observation system 1C will now be described.

Prior to the observation using this system 1C, an operator loads, with the sheath 8, the outer surface of the insertion tube 22P to cover thereof such that the vibration buffering member 22d1 is located close to the objective optical system 22a, the illuminating optical system 22b and the image pickup device 22c and the vibrator 22e2 is located close to the transmission/reception unit 22P.

When the biological observation system 1C is set to the conventional observation mode, the mode switchover circuit 57 enables the first light source unit 3 and the image-signal processing circuit 51 to be active and enables the transmission/reception unit 22P, the signal processing circuit 54, and the vibrating-member control unit 7 to be inactive. Thus the light emitted from the first light source unit 3 is radiated onto the body tissue LT via the first light guide cable 21a and the illuminating optical system 22b.

Thus, in the field of view of the objective optical system 22a, surface images of the body tissue LT is displayed by the monitor 6 in the same manner as described before.

In contrast, when the biological observation system 1C is switched to the ultrasound modulated optical tomography mode, the mode switchover circuit 57 enables the transmission/reception unit 22P, the signal processing circuit 54, and the vibrating-member control unit 7 to be active, instead. The operations of the fist light source unit 3 and the image-signal processing circuit 51 are stopped. In this case, the light emitted from the transmission/reception unit 22P is radiated onto the body tissue LT.

In the ultrasound modulated optical tomography mode, the vibrating-member control unit 7 controls the vibrator 22e2 such that the vibrator 22e2 two-dimensionally vibrates by predetermined amounts of displacement in, for example, the X-axis and Z-axis directions shown in Fig. 7.

The vibration of the vibrator 22e2 permits the not-shown ultrasound transducer in the transmission/reception unit 22P to vibrate together with the vibrator. The ultrasound waves converged by the not-shown acoustic lens in the unit 22P are radiated to the body tissue LT in the X-axis direction in Fig. 7. Hence, the ultrasound waves from the unit 22P is propagated through a region being examined within the body tissue LT, where the region corresponds to the widths of vibration of the vibrator 22e2.

The propagated ultrasound waves produce a denser sound-pressure region within the body tissue LT. Thus the light radiated in the X-axis direction from the transmission/reception unit 22P is reflected (at least partly reflected) at the denser sound-pressure region to return, as reflected light with modulated components, to the unit 22P.

The above vibration operation and the reflected-light detection are performed at intervals, resulting in that a region corresponding to the vibration widths of the vibrator 22e2 in the body tissue LT is scanned.

The reflected light returned to the transmission/reception unit 22P is transmitted to the modulated light/scattered light detecting circuit 53 in the controller 5A via the second light guide cable 21b. Thus in the controller 5A, the same processes as the foregoing are applied to the reflected light.

The monitor 6 represents images such as tomographic images of the inside of the body tissue LT depending modulation/scattering characteristic information to be acquired from the reflected light in the same way as the foregoing.

In this way, in the biological observation system 1C of the present embodiment, the modulation/scattering characteristic information can be displayed as images by loading, with the sheath 8, the insertion tube of the endoscope with the sheath 8 positionally adjusted in place. Thus it is possible for the present embodiment to provide the advantages the similar to those in the foregoing embodiments. In addition, the sheath 8 is equipped with a vibration source (vibrator 22e2). Thus the distal end 22D of the insertion tube 21 can be vibrated from the outside of the distal end 22D in order to perform the two-dimensional or three-dimensional scan. It is not required to arrange the vibration source within the distal end 22D, so that the structure of the distal end 22 itself can be simplified.

Incidentally, in the foregoing biological observation system 1C, the transmission/reception unit 22P may be configured to radiate the light in the Z-axis direction in Fig. 7. The vibrator 22e2 may have a through-hole through which the light passes.

Although the descriptions above contain many specificities, these should not be construed as limiting the scope of the invention but as merely providing illustrations of some of the presently preferred embodiments of the present invention. Thus the scope of the present invention should be determined by the appended claims.

## Claims

1. An endoscope comprising:
an ultrasound generating member (22g, 22P) that generates ultrasound waves toward a region to be examined of an object;
a light transmitting/receiving member (22i, 21b, 22j, 22P, 22l, 22m) that radiates light from a light source toward the region to be examined and receives light reflected by a region of the ultrasound waves within the region to be examined; and
a moving member (22e, 22e1, 59a, 22e2, 8) that spatially moves both the ultrasound generating member and the light transmitting/receiving member together as a unitary member.

2. The endoscope of claim 1, comprising a light converging member (22n, 220) that converges the light in a direction in which the region to be examined resides.

3. The endoscope of claim 1, comprising an ultrasound converging member (22h) that converges the ultrasound waves in a direction in which the region to be examined resides.

4. The endoscope of claim 3, comprising an adaptor (22f) detachably secured to a distal end of an insertion tube of the endoscope, wherein the ultrasound generating member, the ultrasound converging member, and the light transmitting/receiving member are arranged within the adaptor.

5. The endoscope of claim 1, wherein the moving member is a vibrator (22e, 22e1, 22e2, 8) that spatially vibrates both the ultrasound generating member and the light transmitting/receiving member together as the unitary member.

6. The endoscope of claim 1, wherein the moving member is a rotating member (59a) that spatially rotates both the ultrasound generating member and the light transmitting/receiving member together as the unitary member.

7. A medical apparatus comprising:
an ultrasound generating member (22g, 22P) that generates ultrasound waves toward a region to be examined of an object;
a light source unit (4) that emits light reaching the region to be examined;
a light transmitting/receiving member (22i, 21b, 22j, 22P, 22l, 22m) that radiates light from a light source unit toward the region to be examined and receives light reflected by a region of the ultrasound waves within the region to be examined; and
a moving member (22e, 22e1, 59a, 22e2, 8) that spatially moves both the ultrasound generating member and the light transmitting/receiving member together as a unitary member.

8. The medical apparatus of claim 7, comprising a light converging member (22n, 22o) that converges the light in a direction in which the region to be examined resides.

9. The medical apparatus of claim 7, comprising an ultrasound converging member (22h) that converges the ultrasound waves in a direction in which the region to be examined resides.

10. The medical apparatus of claim 7, comprising a video signal producer (5, 5A) that estimates information indicating at least one of modulation and scattering of the reflected light which have undergone in the region to be examined.

11. The medical apparatus of claim 7, wherein the ultrasound generating member, the light transmitting/receiving member, and the moving member are arranged in an insertion tube of the endoscope.

12. The medical apparatus of claim 11, wherein at least the ultrasound generating member and the light transmitting/receiving member are arranged in a distal end of the insertion tube of the endoscope.

13. The medical apparatus of claim 12, wherein the moving member is composed of a rotating member (59a) that spatially rotates both the ultrasound generating member and the light transmitting/receiving member together as the unitary member in a state where a direction along which the ultrasound waves are radiated toward the region and a direction along which the light is emitted toward the region agree with each other.

14. The medical apparatus of claim 13, wherein the ultrasound generating member and the light transmitting/receiving member are mutually unified such that the direction along which the ultrasound waves are radiated toward the region and the direction along which the light is emitted toward the region are perpendicular to a longitudinal direction of the distal end of the insertion tube of the endoscope.

15. The medical apparatus of claim 11, wherein the ultrasound generating member, the light transmitting/receiving member, and the moving member are arranged in a distal end of the insertion tube of the endoscope.

16. The medical apparatus of claim 15, wherein the moving member is composed of a vibrator (22e, 22e1, 22e2, 8) that spatially vibrates both the ultrasound generating member and the light transmitting/receiving member together as the unitary member in a state where a direction along which the ultrasound waves are radiated toward the region and a direction along which the light is emitted toward the region agree with each other.

17. The medical apparatus of claim 16, wherein the ultrasound generating member and the light transmitting/receiving member are mutually unified such that the direction along which the ultrasound waves are radiated toward the region and the direction along which the light is emitted toward the region are along a longitudinal direction of the distal end of the insertion tube of the endoscope.

18. The medical apparatus of claim 16, wherein the ultrasound generating member and the light transmitting/receiving member are mutually unified such that the direction along which the ultrasound waves are radiated toward the region and the direction along which the light is emitted toward the region are perpendicular to a longitudinal direction of the distal end of the insertion tube of the endoscope.

19. The medical apparatus of claim 16, wherein the ultrasound generating member, the light transmitting/receiving member, and the vibrator are embedded in the distal end of the insertion tube of the endoscope.

20. The medical apparatus of claim 16, wherein the vibrator is disposed outside of the distal end of the insertion tube of the endoscope.

21. A medical apparatus comprising:
an ultrasound generating member (22g, 22P) that generates ultrasound waves toward a region to be examined of an object;
a light source unit (4) that emits light reaching the region to be examined;
a light transmitting/receiving member (22i, 21b, 22j, 22P, 22l, 22m) that radiates light from a light source unit toward the region to be examined and receives light reflected by a region of the ultrasound waves within the region to be examined; and
a moving member (22e, 22e1, 59a, 22e2, 8) that spatially moves the ultrasound generating member.

22. The medical apparatus of claim 21, comprising a light converging member (22n, 22o) that converges the light in a direction in which the region to be examined resides.

23. The medical apparatus of claim 21, comprising an ultrasound converging member (22h) that converges the ultrasound waves in a direction in which the region to be examined resides.

24. The medical apparatus of claim 21, comprising a video signal producer (5, 5A) that estimates information indicating at least one of modulation and scattering of the reflected light which have undergone in the region to be examined.

25. The medical apparatus of claim 21, wherein the ultrasound generating member, the light transmitting/receiving member, and the moving member are arranged in an insertion tube of the endoscope.

26. The medical apparatus of claim 25, wherein at least the ultrasound generating member and the light transmitting/receiving member are arranged in a distal end of the insertion tube of the endoscope.

27. The medical apparatus of claim 26, wherein the moving member is composed of a rotating member (59a) that spatially rotates both the ultrasound generating member and the light transmitting/receiving member together as the unitary member in a state where a direction along which the ultrasound waves are radiated toward the region and a direction along which the light is emitted toward the region agree with each other.

28. The medical apparatus of claim 27, wherein the ultrasound generating member and the light transmitting/receiving member are mutually unified such that the direction along which the ultrasound waves are radiated toward the region and the direction along which the light is emitted toward the region are perpendicular to a longitudinal direction of the distal end of the insertion tube of the endoscope.

29. The medical apparatus of claim 25, wherein the ultrasound generating member, the light transmitting/receiving member, and the moving member are arranged in a distal end of the insertion tube of the endoscope.

30. The medical apparatus of claim 29, wherein the moving member is composed of a vibrator (22e, 22e1, 22e2, 8) that spatially vibrates both the ultrasound generating member and the light transmitting/receiving member together as the unitary member in a state where a direction along which the ultrasound waves are radiated toward the region and a direction along which the light is emitted toward the region agree with each other.

31. The medical apparatus of claim 30, wherein the ultrasound generating member and the light transmitting/receiving member are mutually unified such that the direction along which the ultrasound waves are radiated toward the region and the direction along which the light is emitted toward the region are along a longitudinal direction of the distal end of the insertion tube of the endoscope.

32. The medical apparatus of claim 30, wherein the ultrasound generating member and the light transmitting/receiving member are mutually unified such that the direction along which the ultrasound waves are radiated toward the region and the direction along which the light is emitted toward the region are perpendicular to a longitudinal direction of the distal end of the insertion tube of the endoscope.

33. The medical apparatus of claim 30, wherein the ultrasound generating member, the light transmitting/receiving member, and the vibrator are embedded in the distal end of the insertion tube of the endoscope.

34. The medical apparatus of claim 30, wherein the vibrator is disposed outside of the distal end of the insertion tube of the endoscope.
